# DEMANDE DE BREVET EUROPEEN

(11) **EP 4 052 575 A1**
(43) Date de publication de la demande: **07.09.2022**
(21) Numéro de dépôt: 22305245.7
(22) Date de dépôt: 03.03.2022
(51) Int. Cl.: A01N 43/16, A01N 65/08, A01P 1/00

(54) **UTILISATION D'UN EXTRAIT DE RAISIN EN TANT QUE VIRUCIDE A L'ENCONTRE DES VIRUS DE LA FAMILLE DES CORONAVIRUS**

(30) Priorité: 03.03.2021 FR 2102076
(71) Demandeur: Berkem Developpement, 33290 Blanquefort (FR)
(72) Inventeur: MESSAOUDI, Daouia, 69800 SAINT-PRIESST (FR); FAHY, Olivier, 33000 BORDEAUX (FR); PERON, Jean-Louis, 24680 LAMONZIE SAINT MARTIN (FR); NKILIZA, Jean, 24230 SAINT ANTOINE DE BREUILH (FR)
(74) Mandataire: Plasseraud IP

(57) **Abrégé**

La présente invention est du domaine de la désinfection et de l'antisepsie et concerne plus particulièrement l'utilisation d'un extrait de raisin en tant que virucide à l'encontre des virus de la famille des coronavirus, des adénovirus, des norovirus murins, des virus de la vaccine et des poliovirus.

## Description

### Domaine technique

La présente invention est du domaine de la désinfection et de l'antisepsie et concerne plus particulièrement l'utilisation d'un extrait de raisin en tant que virucide à l'encontre des virus de la famille des coronavirus, des adénovirus, des norovirus murins, du virus de la vaccine et des poliovirus.

### Technique antérieure

Les produits désinfectants et antiseptiques sont communément utilisés dans une large variété de produits incluant des désinfectants ménagers et/ou industriels mais également des produits destinés au lavage des matières kératiniques et plus particulièrement des mains.

La désinfection se définit comme une méthode utilisée pour inactiver les microorganismes sur une surface inerte (lieu, objet, ...). L'antisepsie elle se définit comme une méthode utilisée pour éliminer les microorganismes sur les tissus vivants telles que la peau, les muqueuses, les plaies.

L'étude de l'activité virucide des produits désinfectants et antiseptiques est primordiale. En effet, si le pouvoir bactéricide des désinfectants et des antiseptiques est bien étudié, l'activité virucide l'est moins. Un désinfectant peut être bactéricide sans pour cela être virucide. Or, dans le contexte de la pandémie de Covid-19, les produits virucides ayant un effet sur les virus de la famille des coronavirus sont d'une part, nécessaires et d'autre part, extrêmement recherchés.

En pratique, le choix d'un produit virucide doit prendre en compte différents critères d'efficacité, d'innocuité (ou de moindre toxicité), d'autant que les produits virucides dans le contexte de la pandémie sont utilisés à très large échelle, sur un très grand nombre d'utilisateurs (enfants, adultes, personnes âgées, ...). Les contraintes sont également relatives à la compatibilité avec les matériels à traiter, mais aussi à l'impact environnemental. Il existe en effet une forte demande pour des désinfectants et antiseptiques d'origine végétale ayant ainsi un faible impact sur la santé et l'environnement. Il est aussi avantageux que le produit virucide puisse avoir un large spectre et soit actif à l'encontre des nombreux virus enveloppés et non enveloppés.

La présente invention porte sur l'utilisation d'un extrait de raisin en tant qu'antiseptique et désinfectant, préférentiellement à l'encontre des virus de la famille des coronavirus. En tant qu'extrait végétal, le produit a avantageusement un faible impact sur la santé et l'environnement.

Dans la littérature, un extrait de pépins de raisin a été testé sur des virus entériques (calicivirus félin, FCV-F9 ; norovirus murin, MNV-1 et bactériophage MS2) ainsi que sur le virus de l'hépatite A (VHA ; souche HM175).

Une réduction significative des quatre virus a été observée et a permis aux auteurs de conclure que l'extrait de pépins de raisin est prometteur pour une application dans l'industrie alimentaire en tant que nouvelle alternative naturelle peu coûteuse pour réduire la contamination virale et améliorer la sécurité alimentaire.

L'activité thérapeutique d'un extrait de pépins de raisin a également été démontré à l'encontre du virus de l'hépatite A et C et du virus respiratoire syncytial (VRS).

Toutefois, le pouvoir antiseptique et désinfectant et plus particulièrement le pouvoir antiseptique et désinfectant à l'encontre des virus de la famille des coronavirus, d'un extrait de raisin n'a jamais été démontré.

Il a de plus été démontré que l'extrait de raisin, outre son effet à l'encontre des virus de la famille des coronavirus, a également un effet à l'encontre des virus de la famille des adénovirus, des norovirus murins, des virus de la vaccine et des poliovirus, offrant ainsi un spectre très large.

Par ailleurs, des critères très spécifiques ont été établis pour démontrer l'activité virucide d'un produit. Ces critères sont les suivants :
- le titre de la suspension d'essai est suffisant pour permettre la réduction de 4 log après traitement avec le produit ;
- la réduction entre le logarithme du titre du témoin viral et celui du virus utilisé lors de l'essai de référence pour l'inactivation du coronavirus humain doit être comprise entre 0,75 et 3,5 après 5 min et entre 2 et 4 après 15 min (référence au virus de la vaccine, seul virus enveloppé obligatoire de la norme)
- la cytotoxicité de la solution d'essai du produit n'affecte pas la morphologie et la croissance cellulaire, ni la sensibilité au virus dans les dilutions du mélange d'essai qui sont nécessaires pour démontrer une réduction de 4 log du titre viral
- lors du contrôle de l'efficacité de l'arrêt de l'activité du produit, la différence de titre avec la suspension d'essai doit être ≤ 0,5 log;
- une concentration au moins par essai doit montrer une réduction de 4 log ou plus et une concentration au moins doit montrer une réduction inférieure à 4 log.

Ces critères sont ceux de la norme EN 14476 de Juillet 2019.

Il est donc nécessaire de mettre au point un désinfectant et un antiseptique, préférentiellement virucide à l'encontre des virus de la famille des coronavirus, des adénovirus, des norovirus murins, des virus de la vaccine et des poliovirus qui soit efficace, réponde aux critères de la norme EN 14476, tout en ayant un faible impact sur la santé et l'environnement.

### Résumé

Il a été démontré par les inventeurs que l'extrait de raisin selon la présente invention présente tous les critères précédemment mentionnés permettant avantageusement de conclure à l'activité virucide dudit extrait de raisin à l'encontre des virus de la famille des coronavirus.

L'extrait de raisin selon la présente invention possède en effet une activité virucide supérieure ou égale à 4.1 log à l'encontre de la souche humaine du coronavirus 229E.

Il a également été avantageusement démontré que l'extrait de raisin selon la présente invention présente également tous les critères précédemment mentionnés permettant de conclure à l'activité virucide dudit extrait à l'encontre des virus enveloppés et non-enveloppés.

En effet, et selon les critères de la norme EN 14476/2019, l'extrait de raisin selon l'invention a une activité virucide (log R ≥ 4) à l'encontre des adénovirus, des norovirus murins, des virus de la vaccine et des poliovirus, soit l'ensemble des virus enveloppés et non enveloppés tels exigés dans cette norme.

Avantageusement toujours, l'extrait selon l'invention étant un extrait végétal, ce produit est efficace tout en ayant un faible impact sur la santé et l'environnement.

Ainsi, la présente invention concerne l'utilisation d'un extrait de raisin en tant que désinfectant à l'encontre des virus de la famille des coronavirus.

La présente invention concerne également l'utilisation d'un extrait de raisin en tant que désinfectant à l'encontre des virus de la famille des adénovirus, des norovirus murins, du virus de la vaccine et des poliovirus.

La présente invention concerne également un procédé de désinfection des instruments, textiles et surfaces.

La présente invention concerne également un procédé d'antisepsie non thérapeutique comprenant l'application sur les matières kératiniques, préférentiellement les mains, d'une composition antiseptique comprenant, dans un milieu physiologiquement acceptable, un extrait de raisin.

La présente invention concerne également une composition antiseptique comprenant, dans un milieu physiologiquement acceptable, un extrait de raisin pour son utilisation thérapeutique en tant qu'antiseptique.

Les inventeurs ont également démontré que l'extrait de raisin selon la présente invention présente un effet activateur, préférentiellement un effet activateur des ammoniums quaternaires ou de leurs sels. Ainsi, la dose efficace en ammoniums quaternaires ou leurs sels est avantageusement réduite, permettant d'offrir des compositions désinfectantes efficaces et présentant une cytotoxicité moindre.

Ainsi, la présente invention concerne également une composition désinfectante comprenant un extrait de raisin et un agent désinfectant choisi parmi un ammonium quaternaire, un sel d'ammonium quaternaire, une amine, un aldéhyde, un phénol, un alcool, l'acide peracétique ou leurs mélanges, ledit extrait de raisin est un extrait de pépins de l'espèce *Vitis vinifera L* et est présent en un teneur d'au moins 1% en poids par rapport au poids total de la composition et l'agent désinfectant est présent en une teneur d'au moins 1% en poids par rapport au poids total de la composition.

### Brève description des dessins

D'autres caractéristiques, détails et avantages apparaîtront à la lecture de la description détaillée ci-après, et à l'analyse des dessins annexés, sur lesquels :
**Fig. 1**

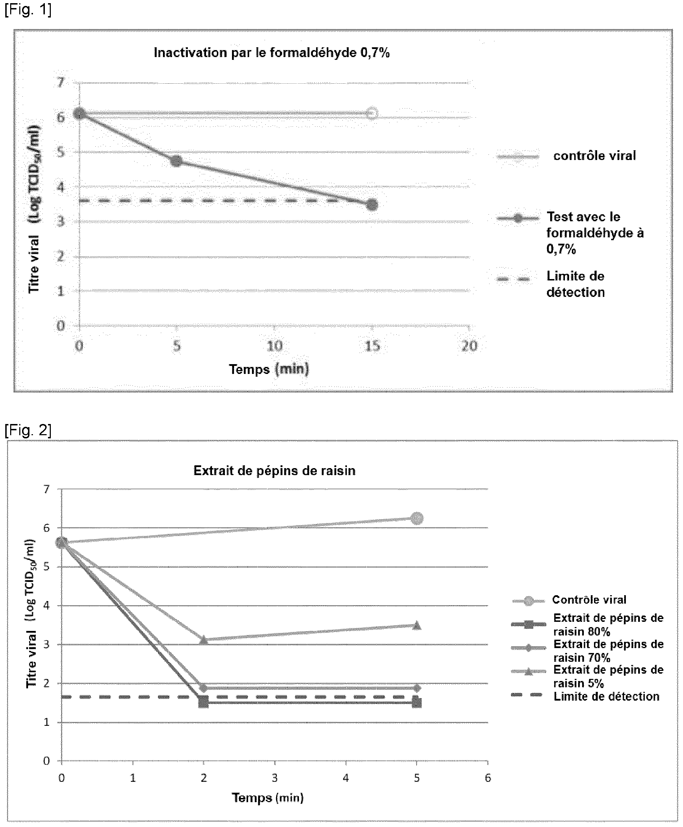

   [Fig. 1] montre le titre viral (log TCID₅₀/mL) d'un échantillon viral traité avec du formaldéhyde à 0,7% en fonction du temps de contact. Il s'agit d'un contrôle interne permettant d'évaluer la sensibilité de l'échantillon viral par rapport à un produit de référence : le formaldéhyde.
**Fig. 2**
   [Fig. 2] montre le titre viral (log TCID₅₀/mL) d'un échantillon viral traité avec un extrait de pépins de raisin selon l'invention, à différentes concentrations (80%, 70% et 5%) en fonction du temps de contact.
**Fig. 3**

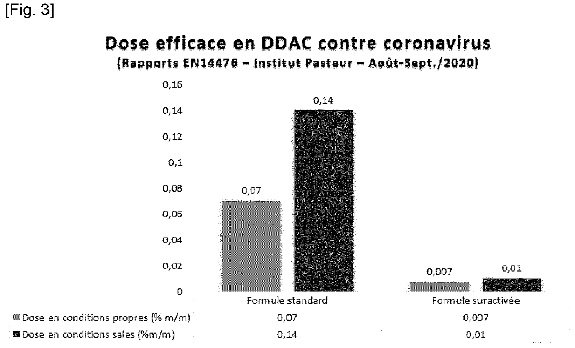

   [Fig. 3] montre la dose efficace en DDAC contre les coronavirus et l'effet activateur de l'extrait de pépins de raisin selon l'invention.

### Description détaillée

La présente invention concerne l'utilisation d'un extrait de raisin en tant que désinfectant à l'encontre des virus de la famille des coronavirus.

Avantageusement, l'extrait selon l'invention présente un spectre virus large à l'encontre de nombreux virus enveloppés et non enveloppés. Outre son pouvoir désinfectant à l'encontre des coronavirus, il l'est également à l'encontre des virus de la famille des adénovirus, des norovirus murins, des virus de la vaccine et des poliovirus.

Ainsi, la présente invention concerne également l'utilisation d'un extrait de raisin en tant que désinfectant à l'encontre des virus choisis parmi les virus de la famille des coronavirus, des adénovirus, des norovirus murins, des virus de la vaccine et des poliovirus.

Selon un mode de réalisation, l'extrait de raisin est utilisé en tant que désinfectant à l'encontre des virus de la famille des coronavirus et en outre en tant que désinfectant à l'encontre des virus de la famille des adénovirus, des norovirus murins, des virus de la vaccine et des poliovirus.

### Extrait de raisin (Vitis vinifera L)

L'extrait de raisin selon la présente invention appartient au genre Vitis et est préférentiellement un extrait de l'espèce *Vitis vinifera L.*

Typiquement, l'extrait de raisin pourra être obtenu à partir des bourgeons, des fleurs, des fruits, des feuilles, des graines, de la peau, des racines, des pépins.

Préférentiellement, l'extrait de raisin est obtenu à partir des pépins.

Préférentiellement, l'extrait de raisin est obtenu à partir des pépins de l'espèce *Vitis vinifera L.*

L'extrait de raisin selon la présente invention comprend une teneur en polyphénols supérieure ou égale à 65%.

Typiquement, les polyphénols sont des oligo-proanthocyanidines (OPC). Préférentiellement, la teneur en oligo-proanthocyanidines (OPC) est supérieure ou égale à 30%.

Dans un mode de réalisation la teneur en oligo-proanthocyanidines (OPC) est supérieure ou égale à 90%, préférentiellement supérieure ou égale à 92%, préférentiellement supérieure ou égale à 95% et de manière encore plus préférée, la teneur en oligo-proanthocyanidines (OPC) est supérieure ou égale supérieure à 99%.

Préférentiellement, la teneur en monomère de catéchines est inférieure à 5% et la teneur en proanthocyanidines dimère B1 est inférieure à 8%.

Selon un mode de réalisation, l'extrait de raisin est fabriqué à partir des pépins de raisin de l'espèce *Vitis vinifera L.* et est obtenu par une succession d'étapes d'extraction, de purification et de séchage.

Les principaux constituants de l'extrait de raisin selon l'invention sont quantifiés par chromatographie liquide haute performance en phase inverse avec détection par absorption ultraviolette.

### Activité virucide

Les critères permettant d'attester de l'activité virucide selon la norme EN 14476 :2019 sont les suivants :
- le titre de la suspension d'essai est suffisant pour permettre la réduction de 4 log après traitement avec le produit ;
- la réduction entre le logarithme du titre du témoin viral et celui du virus utilisé lors de l'essai de référence pour l'inactivation du coronavirus humain doit être comprise entre 0,75 et 3,5 après 5 min et entre 2 et 4 après 15 min (référence au virus de la vaccine, seul virus enveloppé obligatoire de la norme).
- la cytotoxicité de la solution d'essai du produit n'affecte pas la morphologie et la croissance cellulaire, ni la sensibilité au virus dans les dilutions du mélange d'essai qui sont nécessaires pour démontrer une réduction de 4 log du titre viral
- lors du contrôle de l'efficacité de l'arrêt de l'activité du produit, la différence de titre avec la suspension d'essai doit être ≤ 0,5 log;
- une concentration au moins par essai doit montrer une réduction de 4 log ou plus et une concentration au moins doit montrer une réduction inférieure à 4 log.

L'extrait de raisin selon l'invention rempli avantageusement tous ces critères permettant de conclure à l'activité virucide de cet extrait à l'encontre des virus de la famille des coronavirus, ainsi que des adénovirus, des norovirus murins, des virus de la vaccine et des poliovirus.

### Famille des coronavirus

Les coronavirus humains pathogènes peuvent être classifiés de la manière suivante :
- genre alpha coronavirus auquel appartiennent le coronavirus 229E (HCoV-229E) et le coronavirus NL63 (HCoV-NL63);
- genre beta coronavirus : (Beta-CoV or β-CoV) divisé selon les groupes suivants :
   Groupe A comprenant le HCoV-OC43 et le HCoV-HKU1;
   Groupe B/C comprenant les coronavirus humains hautement pathogénique que sont le SARS-Cov-2, le SARS-CoV et le MERS-CoV.

L'extrait de raisin selon la présente invention possède une activité virucide supérieure ou égale à 4.1 log à l'encontre de la souche humaine du coronavirus 229E.

Selon un mode de réalisation, les coronavirus sont choisis parmi les coronavirus du genre alpha coronavirus, les coronavirus du genre beta coronavirus.

Selon un mode de réalisation préféré, les coronavirus sont choisis parmi les coronavirus du genre alpha coronavirus tels que le coronavirus 229E (HCoV-229E) et le coronavirus NL63 (HCoV-NL63), les coronavirus du groupe A du genre beta coronavirus tels que le HCoV-OC43 et le HCoV-HKU1 et les coronavirus du groupe B/C du genre beta coronavirus tels que le SARS-Cov-2, le SARS-CoV et le MERS-CoV.

Selon un mode de réalisation préféré, l'extrait de raisin possède une activité virucide à l'encontre des coronavirus humains hautement pathogénique tels que le SARS-Cov-2, le SARS-CoV et le MERS-CoV, préférentiellement le SARS-Cov-2 et le MERS-CoV, et de manière préférée le SARS-Cov-2.

La souche de coronavirus humain 229E est une souche endémique, connue, et facilement manipulable en laboratoire de classe II, à l'inverse du SARS-CoV-2 qui se manipule en laboratoire de classe III actuellement. Le Haut Conseil de Santé publique a émis différents avis et a pris en compte pour établir ses préconisation les données décrites avec la souche endémique de coronavirus humain 229E. Typiquement, la survie du SARS-CoV-2 dans l'environnement pourrait s'apparenter à celle d'autres coronavirus humains comme le SARS-CoV et le MERS-CoV et l'efficacité des procédures de désinfection sur les coronavirus humains tels que les SARS-CoV ou MERS-CoV devrait être similaire pour SARS-CoV-2.

Il est également rappelé que la plupart des données ont été décrites avec la souche endémique du coronavirus humain (HCoV-229E). Les données obtenues sur HCoV-229E pourraient donc s'extrapoler aux virus de la famille des coronavirus.

### Microorganismes additionnels : Familles des poliovirus, norovirus murin, adenovirus et virus de la vaccine

Avantageusement, l'extrait de raisin selon l'invention possède une activité virucide à l'encontre de microorganismes additionnels tels que les poliovirus, les norovirus murins, les adenovirus et le virus de la vaccine.

Les inventeurs ont avantageusement démontré que l'extrait de raisin selon l'invention possède une activité virucide à l'encontre des adénovirus, des norovirus murins, des virus de la vaccine et des poliovirus.

En effet, et selon les critères de la norme EN 14476/2019, l'extrait de raisin selon l'invention a une activité virucide (log R ≥ 4) à l'encontre des adénovirus, des norovirus murins, des virus de la vaccine et des poliovirus, soit l'ensemble des virus enveloppés et non enveloppés tels exigés dans cette norme.

### Désinfectant

Un procédé de désinfection a pour but d'éliminer les microorganismes et/ou d'inactiver le ou les virus sur les surfaces inertes tels que les sols, les mobiliers, les objets. Cette opération permet de supprimer jusqu'à 99% des microorganismes.

Ainsi, un désinfectant est un produit permettant d'éliminer les microorganismes et/ou d'inactiver le ou les virus sur les surfaces inertes tels que les sols, les mobiliers, les objets, les instruments.

Compte-tenu de son activité virucide, l'extrait de raisin selon l'invention est avantageusement utilisé en tant que désinfectant à l'encontre des virus de la famille des coronavirus, mais également à l'encontre des virus de la famille des adénovirus, des norovirus murins, des virus de la vaccine et des poliovirus.

### Procédé de désinfection

L'invention concerne également un procédé de désinfection d'une surface au moyen d'une composition désinfectante comprenant un extrait de raisin, le procédé comprenant les étapes consistant à appliquer ladite composition sur ladite surface.

On entend par surface, toute surface du milieu hospitalier, de l'industrie agro-alimentaire, de magasins, de restaurants, de l'espace public ou même des surfaces chez les particuliers. Ainsi, la surface à désinfecter au sens de la présente invention s'entend de manière large et s'applique à toute surface susceptible d'entrer en contact avec un patient et/ou le personnel médical et/ou des surfaces touchées par des personnes différentes et/ou des denrées alimentaires, .... On citera, à titre illustratif la surface d'un sol, d'un instrument, d'un objet, d'un mobilier, d'un matériau, d'un plan de travail, d'un matériel ou d'un élément présent dans les transports en commun, un mobilier public, un outil de transport tels qu'un container ou tout autre surface nécessitant d'être désinfectée.

L'homme du métier respectera le temps de contact nécessaire pour que la composition agisse.

Le temps de contact est un temps après application suffisamment long pour assurer la destruction des microorganismes, préférentiellement des virus choisis parmi les familles des coronavirus, des adénovirus, des norovirus murins, des virus de la vaccine, des poliovirus, et préférentiellement les virus de la famille des coronavirus, susceptibles d'être présents sur cette surface. Typiquement, le temps de contact est d'au moins trente secondes, préférentiellement au moins une minute, préférentiellement au moins deux minutes, préférentiellement d'au moins cinq minutes, à température ambiante.

Typiquement l'application sera réalisée par voie aérienne ou par application mécanique.

Typiquement, l'application par voie aérienne sera réalisée via un aérosol ou un fumigène.

Typiquement, l'application par voie mécanique sera réalisée par pulvérisation, par application de lingettes imprégnées de la composition désinfectante, par inondation.

Selon un mode de réalisation, le procédé selon l'invention comprend une étape supplémentaire d'élimination de la composition désinfectante de ladite surface.

Typiquement, l'élimination de ladite composition est réalisée par rinçage, par exemple à l'eau claire ou par essuyage, sec ou humide.

Selon un mode de réalisation, l'invention concerne également un procédé de désinfection d'instrument au moyen d'une composition désinfectante comprenant un extrait de raisin, le procédé comprenant les étapes consistant à immerger l'instrument à désinfecter dans ladite composition désinfectante.

On entend par instrument, tout instrument du milieu hospitalier, de l'industrie agro-alimentaire, de magasins, de restaurants, de l'espace public, ou même des instruments chez les particuliers. Ainsi, l'instrument à désinfecter au sens de la présente invention s'entend de manière large et s'applique à tout instrument susceptible d'entrer en contact avec un patient et/ou le personnel médical et/ou des instruments touchées par des personnes différentes et/ou des denrées alimentaires, .... On citera, à titre illustratif, les instruments médicaux, les outils de transport tels que des containers.

L'homme du métier respectera le temps d'immersion nécessaire pour que la composition agisse.

Le temps d'immersion est un temps suffisamment long pour assurer la destruction des microorganismes, préférentiellement des virus de la famille des coronavirus, susceptibles d'être présents sur l'instrument à désinfecter. Typiquement, le temps d'immersion est d'au moins une minute, préférentiellement au moins deux minutes, préférentiellement au moins 5 minutes, préférentiellement au moins 10 minutes, préférentiellement au moins 15 minutes, préférentiellement au moins 20 minutes, préférentiellement au moins 25 minutes, préférentiellement au moins 30 minutes, préférentiellement au moins 40 minutes, préférentiellement au moins 45 minutes, préférentiellement au moins 50 minutes et de manière préférée au moins soixante minutes, à température ambiante.

Selon un mode de réalisation, le procédé selon l'invention comprend une étape supplémentaire de rinçage, par exemple à l'eau claire.

L'invention concerne également un procédé de désinfection d'un textile au moyen d'une composition désinfectante comprenant un extrait de raisin, le procédé comprenant les étapes consistant à appliquer ladite composition sur ledit textile ou immerger ledit textile dans ladite composition désinfectante.

On entend par textile, tout textile du milieu hospitalier, de l'industrie agro-alimentaire, de magasins, de restaurants, de l'espace public tel que des textiles présents dans les transports en commun, ou même des textiles chez les particuliers. Ainsi, le textile à désinfecter au sens de la présente invention s'entend de manière large et s'applique à tout textile susceptible d'entrer en contact avec un patient et/ou le personnel médical et/ou des denrées alimentaires et/ou des textiles touchées par des personnes différentes et/ou des textiles de la vie quotidienne.

L'homme du métier respectera le temps de contact ou le temps d'immersion nécessaire pour que la composition agisse.

Le temps de contact est un temps après application suffisamment long pour assurer la destruction des microorganismes, préférentiellement des virus choisis parmi les familles des coronavirus, des adénovirus, des norovirus murins, des virus de la vaccine, des poliovirus, et préférentiellement les virus de la famille des coronavirus,, susceptibles d'être présents sur le textile à désinfecter. Typiquement, le temps de contact est d'au moins une minute, préférentiellement au moins deux minutes, préférentiellement au moins cinq minutes, préférentiellement au moins 10 minutes, préférentiellement au moins 15 minutes, et de manière préférée au moins vingt minutes, à température ambiante.

Typiquement, le temps d'immersion est d'au moins une minute, préférentiellement au moins deux minutes, préférentiellement au moins 5 minutes, préférentiellement au moins 10 minutes, préférentiellement au moins 15 minutes, préférentiellement au moins 20 minutes, préférentiellement au moins 25 minutes, préférentiellement au moins 30 minutes, préférentiellement au moins 40 minutes, préférentiellement au moins 45 minutes, préférentiellement au moins 50 minutes et de manière préférée au moins soixante minutes, à température ambiante.

Selon un mode de réalisation, le procédé selon l'invention comprend une étape supplémentaire de rinçage, par exemple à l'eau claire.

### Composition désinfectante

La composition désinfectante comprend, dans un milieu aqueux ou hydroalcoolique, un extrait de pépins de raisin selon l'invention.

Selon un mode de réalisation, la teneur en extrait de raisin est d'au moins 0,2% en poids par rapport au poids total de la composition, préférentiellement d'au moins 0,5% en poids par rapport au poids total de la composition, préférentiellement au moins 1% en poids par rapport au poids total de la composition, préférentiellement au moins 2% en poids par rapport au poids total de la composition, préférentiellement au moins 3% en poids par rapport au poids total de la composition, préférentiellement au moins 4% en poids par rapport au poids total de la composition, préférentiellement au moins 5% en poids par rapport au poids total de la composition.

Selon un mode de réalisation, la composition désinfectante comprendra en outre au moins un agent choisi parmi les surfactants non ioniques, les anti-mousses, les surfactants anioniques, les polymères hydrosolubles, les agents chélatants, les agents de traitements de surface métallique, les acides, les conservateurs, les stabilisateurs, les produits à effet optique, les enzymes, les cires, les émulsions cireuses, les agents désincrustants, les tensioactifs cationiques, les désinfectants.

Parmi les surfactants non ioniques on citera les alcools éthoxylés, les alkylpolyglucosides, les ammonium éthoxylées, les surfactants non ioniques peu moussants, les polyalkylène glycols.

Parmi les surfactants anioniques on citera les éthersulfates d'alcool gras, les sulfates d'alcool gras, les sulfonates d'alkyl benzène linéaire.

Parmi les polymères hydrosolubles on citera les agents dispersants, les épaississants.

Parmi les agents de traitement de surface métallique, on citera les inhibiteurs de corrosion.

Parmi les conservateurs, on citera le Bronopol, l'acide formique, le glutaraldéhyde, le glyoxal, le n-propanol, le phénoxyéthanol, le Dichlorohydroxydiphenylether (DCPP).

Parmi les produits à effet optique et les stabilisateurs on citera les antioxydants, les agents bloquants excités (ESQ^{™}) (stabilisants UV), les azurants optiques, les stabilisateurs UV,

Parmi les enzymes on citera les cellulases, les protéases.

Parmi les agents désincrustants on citera les phosphonates ou les polyphosphonates.

Parmi les désinfectants, on citera les ammoniums quaternaires, les sels d'ammonium quaternaire, les aminés, les aldéhydes, les phénols tels que l'orthophénylphénol, les alcool, l'acide peracétique ou leurs mélanges,

### Effet activateur de l'extrait de raisin

Selon un mode de réalisation, l'extrait de raisin selon l'invention est combiné avec un agent désinfectant choisi parmi un ammonium quaternaire, un sel d'ammonium quaternaire, une amine, un aldéhyde, un phénol tel que l'orthophénylphénol, un alcool, l'acide peracétique ou leurs mélanges.

Parmi les ammonium quaternaires et les sels d'ammonium quaternaires, on peut citer le chlorure d'alkyldiméthylbenzylammonium (ADBAC, BKC), le cation tétraméthylammonium (TMAC), le N-(3-aminopropyl)-N-dodecylpropane-1,3-diamine (diamine), le propionate de N,N-Didecyl-N-methyl-poly(oxyethyl) ammonium (Bardap26), le chlorure de didécyldiméthylammonium (DDAC).

Il a été en effet démontré que l'extrait de raisin selon l'invention a un effet activateur des ammonium quaternaires et/ou des sels d'ammonium quaternaires et permet ainsi de diminuer la dose des ammonium quaternaires et/ou des sels d'ammonium quaternaires dans la composition désinfectante.

Avantageusement, la diminution de la quantité des ammonium quaternaires et/ou des sels d'ammonium quaternaires permet d'obtenir une composition désinfectante efficace et présentant une cytotoxicité moindre.

Préférentiellement, l'extrait de raisin est combiné avec le chlorure de didécyldiméthylammonium (DDAC).

Ainsi, la présente invention concerne également une composition désinfectante comprenant un extrait de raisin, un ammonium quaternaire et/ou un sel d'ammonium quaternaire, ledit extrait de raisin étant présent en un teneur d'au moins 1% en poids par rapport au poids total de la composition et l'ammonium quaternaire et/ou le sel d'ammonium quaternaire est choisi parmi le chlorure d'alkyldiméthylbenzylammonium (ADBAC, BKC), le cation tétraméthylammonium (TMAC), le N-(3-aminopropyl)-N-dodecylpropane-1,3-diamine (diamine), le propionate de N,N-Didecyl-N-methyl-poly(oxyethyl) ammonium (Bardap26), le chlorure de didécyldiméthylammonium (DDAC) et est présent en une teneur d'au moins 1% en poids par rapport au poids total de la composition.

La présente invention concerne également une composition désinfectante comprenant un extrait de raisin et un sel d'ammonium quaternaire, ledit extrait de raisin est un extrait de pépins de l'espèce *Vitis vinifera L* et est présent en un teneur d'au moins 1% en poids par rapport au poids total de la composition et le sel d'ammonium quaternaire est le chlorure de didécyldiméthylammonium et est présent en une teneur d'au moins 1% en poids par rapport au poids total de la composition.

### Antiseptique

Un procédé d'aseptisation s'applique à la désinfection des tissus vivants : peau, cuir chevelu, phanères, muqueuses et plaies. Les produits concernés sont à usage externe, préventif ou curatif. Ainsi, un antiseptique est un produit permettant de désinfecter des tissus vivants.

### Procédé d'antisepsie : non thérapeutique

Ainsi, la présente invention concerne un procédé d'antisepsie non thérapeutique, caractérisé en ce qu'il comprend l'application sur les matières kératiniques d'une composition antiseptique comprenant, dans un milieu physiologiquement acceptable, un extrait de raisin.

Typiquement, les matières kératiniques seront la peau, le cuir chevelu, les phanères, et les muqueuses, préférentiellement la peau et tout préférentiellement la peau des mains.

Typiquement, l'application durant le procédé d'antisepsie de la composition antiseptique peut être réalisée pour le traitement hygiénique des mains par friction ou le lavage hygiénique des mains.

### Composition antiseptique : thérapeutique

Selon un mode de réalisation, la présente invention concerne un procédé thérapeutique d'antisepsie des plaies.

Ainsi, la présente invention concerne un extrait de raisin pour son utilisation dans une méthode thérapeutique de désinfection des matières kératiniques.

La présente invention concerne également une composition antiseptique comprenant, dans un milieu physiologiquement acceptable un extrait de raisin, pour son utilisation dans une méthode thérapeutique de désinfection des matières kératiniques.

Typiquement, la méthode thérapeutique de désinfection s'applique aux plaies des matières kératiniques, préférentiellement la peau et les muqueuses.

La présente invention concerne également l'utilisation d'une composition antiseptique comprenant, dans un milieu physiologiquement acceptable, un extrait de raisin pour la fabrication d'un médicament destiné à la désinfection des matières kératiniques.

La présente invention concerne également l'utilisation d'une composition antiseptique comprenant, dans un milieu physiologiquement acceptable, un extrait de raisin pour la désinfection des matières kératiniques.

La présente invention concerne également une méthode d'antisepsie comprenant l'application sur les matières kératiniques d'un patient, d'une composition antiseptique comprenant, dans un milieu physiologiquement acceptable, un extrait de raisin.

Le terme « physiologiquement acceptable » désigne un milieu qui ne présente pas d'effets secondaires délétères et en particulier qui ne produit pas de rougeurs, d'inflammation, d'échauffement, de tiraillements ou de picotements inacceptables pour l'utilisateur du produit. Le milieu est ainsi compatible avec les matières kératiniques d'êtres humains.

La composition antiseptique peut se présenter sous toutes les formes galéniques utilisées dans ce domaine et normalement utilisées pour une application topique antiseptique, telles que des solutions aqueuses, hydroalcooliques ou huileuses, des solutions ou dispersions du type lotion ou sérum, des émulsions de consistance liquide ou semi-liquide du type lait, obtenues par dispersion d'une phase grasse dans une phase aqueuse (H/E) ou inversement (E/H), des suspensions ou des émulsions, de consistance molle, semi-solide ou solide, du type crème, de gel aqueux ou anhydre, des compositions anhydres, des microparticules ou des dispersions vésiculaires de type ionique et/ou non ionique. Ces compositions sont préparées par les méthodes usuelles connues de l'homme du métier.

Selon un mode de réalisation, les compositions selon l'invention se présentent sous forme de solution monophasique aqueuse, sous forme de solution monophasique hydroalcoolique ,sous forme de gel, hydroalcoolique ou aqueux, ou sous forme d'émulsion huile-dans-eau ou eau-dans huile, préférentiellement huile-dans-eau. On entend par solution monophasique aqueuse, une composition comprenant une phase continue aqueuse comprenant de l'eau et optionnellement, un ou plusieurs solvants organiques miscible à l'eau.

On entend par solution monophasique hydroalcoolique, une composition comprenant une phase hydroalcoolique continue comprenant au moins un alcool, et selon un aspect, plusieurs alcools miscibles entre eux.

On citera, parmi les formes galéniques, sous forme de compositions monophasique aqueuse ou hydroalcoolique convenant à une application topique antiseptique des matières kératiniques, préférentiellement la peau, les gels douche, les nettoyants liquides, les lotions, les gels.

Typiquement, ces formes galéniques pourront être utilisées pour le traitement hygiénique des mains par friction ou le lavage hygiénique des mains.

On entend par émulsion des compositions comportant une phase aqueuse et une phase huileuse dispersées l'une dans l'autre, par exemple des émulsions eau-dans-huile (E/H) ou huile-dans-eau (H/E) ou multiple (E/H/E ou H/E/H)

Selon un mode de réalisation, la composition se présente sous forme d'émulsion eau-dans-huile.

Les émulsions eau-dans-huile (E/H) comportent une phase aqueuse dispersée dans une phase huileuse. Ces émulsions comportent une phase continue huileuse.

Selon un mode de réalisation, la composition se présente sous forme d'émulsion huile-dans-eau. Ces émulsions comportent une phase huileuse dispersée dans une phase aqueuse. Ces émulsions comportent une phase continue aqueuse.

On citera, parmi les formes galéniques sous forme d'émulsions huile-dans-eau ou eau-dans-huile les crèmes, les crèmes lavantes, les mousses, les laits.

Typiquement, ces formes galéniques pourront être utilisées pour le traitement hygiénique des mains par friction ou le lavage hygiénique des mains, mais également, lorsqu'elle est présente sous forme de crème, pour le soin des mains abimées par exemple par des lavages fréquents. La crème antiseptique possédera un pouvoir antiseptique et avantageusement un pouvoir réparateur par la présence de composés hydratants.

Selon un mode de réalisation, les compositions selon l'invention sont sous forme de gel, afin, par exemple, de permettre leur distribution soit par versement à partir d'un flacon, soit par pression d'un tube souple, ou par pression exercée sur la pompe d'un flacon pompe.

La composition désinfectante selon l'invention comprend en outre tout additif pharmaceutiquement acceptable.

On entend par « pharmaceutiquement acceptable » une substance qui n'est pas biologiquement ou autrement indésirable, c'est-à-dire qui peut être incorporée dans une composition pharmaceutique administrée à un patient sans causer d'effets biologiques indésirables ou sans interagir de manière délétère avec l'un des autres composants de la composition dans laquelle elle est contenue, par exemple en inhibant ou diminuant les propriétés antivirales de l'extrait de raisin selon l'invention. Lorsque le terme « pharmaceutiquement acceptable » est utilisé pour désigner un support ou un excipient pharmaceutique, il est sous-entendu que le support ou l'excipient satisfait aux normes requises en matière de tests toxicologiques et de fabrication.

Typiquement, l'excipient pharmaceutiquement acceptable pourra être choisi parmi un humectant, un diluant, un désintégrant, un liant, un agent de glissement, un agent lubrifiant, un agent mouillant, un agent tampon, un agent de suspension, un adjuvant, un émulsifiant, un absorbant, un conservateur, un agent de surface, un antioxydant, une huile, une cire, un agent gélifiant, un agent émulsionnant, un tensioactif ou un mélange de ceux-ci.

### Humectant

La composition selon l'invention peut comprendre au moins un humectant.

L'humectant pourra être choisi parmi les polyols et/ou les esters d'acides gras et de polyéthylène glycol.

Par « polyols », on entend toute molécule présentant dans sa structure au moins deux groupements hydroxy (-OH) libres. Ces polyols sont de préférence liquides à température ambiante (25°C).

Typiquement, le polyol sera choisi parmi le maltitol, le mannitol, le xylitol, l'érythritol, le sorbitol, l'isosorbide, le glycérol ou la glycérine, le glucose, le saccharose, le polydextrose, les sirops de glucose hydrogénés, les dextrines, les maltodextrines, les sirops de glucose, et leurs mélanges.

Selon un mode de réalisation, le polyol est la glycérine.

### Huile

La composition selon l'invention comprend au moins une huile.

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Selon un mode de réalisation, l'huile sera choisie parmi les huiles volatiles, les huiles non volatiles et leurs mélanges.

La phase grasse liquide comprend avantageusement une ou plusieurs huiles non volatiles qui procurent un effet émollient sur la peau.

Parmi les huiles volatiles, on peut citer les esters gras, les benzoates d'alcools en C12 à C15, les esters de glycol, les acides gras supérieurs tels que l'acide myristique, l'acide palmitique, l'acide stéarique, l'acide béhénique, l'acide oléique, l'acide linoléique, l'acide linolénique ou l'acide isostéarique.les huiles végétales comme l'huile d'avocat, l'huile de camélias, l'huile de noisette, l'huile de tsubaki, l'huile de noix de cajou, l'huile d'argan, l'huile de soja, l'huile de pépins de raisin, l'huile de sésame, l'huile de maïs, l'huile de germes de blé, l'huile de colza, l'huile de tournesol, l'huile de coton, l'huile de jojoba, l'huile d'arachide, l'huile de macadamia, l'huile d'amande douce, l'huile d'olive et leurs mélanges, les beurres végétaux comme le beurre de karité, le beurre de camellia, les huiles minérales

Ces huiles non volatiles peuvent également être des huiles de type hydrocarbures ou siliconées telles que l'huile de paraffine, de squalane, la vaseline, les diméthyls siloxanes et leurs mélanges.

Typiquement, l'huile pourra être une huile minérale.

### Cire

Par « cire », on entend un corps gras à changement d'état liquide / solide réversible, ayant une température de fusion supérieure à 30°C et généralement inférieure à 90°C, qui est liquide dans les conditions de préparation de la composition et présente à l'état solide une organisation cristalline anisotrope.

Les cires peuvent notamment être choisies parmi les cires animales, les cires végétales et les cires synthétiques ou de silicone renfermant des groupements polaires tels que les esters.

### Agent gélifiant

Par agent gélifiant, on entend un composé qui, en présence d'un solvant, crée des liaisons intermacromoléculaire plus ou moins fortes induisant ainsi un réseau tridimensionnel qui fige ledit solvant.

L'agent gélifiant est choisi parmi les agents gélifiant organiques hydrophiles, et/ou les agents gélifiants organiques liphophiles, et/ou les agents gélifiants organiques amphiphiles, et/ou les agents gélifiant minéraux.

### Liant

On entend par «liant» des composés conférant une cohésion accrue de la composition cosmétique. Cette cohésion peut être ajustée en fonction de la quantité et de l'affinité chimique de l'agent liant par rapport aux ingrédients de la composition cosmétique.

### Agent émulsionnant

La composition peut comprendre un ou plusieurs les émulsionnants huile-dans-eau (H/E) ou eau-dans-huile (E/H).

L'émulsionnant huile-dans-eau (H/E) est un agent émulsionnant de HLB supérieure ou égale à 8 et choisi parmi les esters de sorbitane éventuellement polyéthoxylés, les esters d'acides gras et de glycérol, les esters ou polyesters d'acides gras et de sucrose, les esters d'acides gras et de polyéthylèneglycol, les polysiloxanes modifiés polyéthers, les éthers d'alcools gras et de polyéthylèneglycol, les alkylpolyglycosides et la lécithine hydrogénée, les alcools gras, les esters de sorbitane, les mélanges d'alcool gras et d'esters de phosphate sans que cette liste ne soit limitative, et leurs mélanges.

L'émulsionnant eau-dans-huile (E/H) est un agent émulsionnant de HLB inférieure à 8 et choisi parmi les esters gras non éthoxylés de polyols, et notamment parmi les esters gras non éthoxylés de glycérol, de polyglycérols, de sorbitol, de sorbitan, d'anydrodrohexitols comme en particulier l'isosorbide, de mannitol, de xylitol, d'érythritol, de maltitol, de saccharose, de glucose, de polydextrose, de sirops de glucose hydrogéné, de dextrines et d'amidons hydrolysés.

### Tensioactif

La composition peut comprendre un tensioactif choisi parmi les tensioactifs ioniques, non ioniques, anioniques ou amphotères ou zwitterioniques. Ces tensioactifs sont choisis pour leur fonction détergente et moussante.

### Principes actifs

La composition selon l'invention peut comprendre au moins un autre principe actif. On citera à titre illustratif, un agent antibactérien, un agent antifongique, un agent anti-irritant, un agent antioxydant, un agent anti radicalaire, un agent cicatrisant, un agent hydratant, un agent émollient, des vitamines, des minéraux.

Préférentiellement, la composition comprendra un agent hydratant.

### Exemples

### Exemple 1 : démonstration de l'activité virucide d'un extrait de pépins de raisin selon l'invention à l'encontre des coronavirus

### Conditions expérimentales

Durée du test : du 06/08/2020 au 18/08/2020
Produit de dilution : eau dure
Concentrations testées : 5% (soit 0,2% d'extrait), 70% (soit 2.8% d'extrait) et 80% (soit 3,2% d'extrait). Ces doses sont obtenues après dilution d'une solution d'extrait de raisin à 4% dans l'eau.
Aspect du produit dilué : brun
Temps de contact : 2 et 5 minutes
Température du test : 20°C
Substance interférente : solution d'albumine bovine (BSA) à 0,3 g/L
Souche : souche du coronavirus humain 229E (HCoV-229E)
Lignée cellulaire : cellules Huh-7, DMEM + Glutamax^{™} supplémenté avec 10% de sérum de veau fœtal (FCS) et 1% d'antibiotiques
Incubation : 6 jours à 33°C avec 5%CO₂
Méthode de quantification : plaques de micro-titrage (TCID₅₀/mL avec la méthode de calcul Spearman-Kärber)

### Principe du test

Un échantillon du produit dilué dans de l'eau dure est ajouté à une suspension d'essai de virus, dans une solution d'une substance interférente. Le mélange est maintenu à 20°C pendant 2 et 5 minutes. Une partie aliquote est prélevée à l'issue de ces temps de contact. L'activité virucide est immédiatement supprimée selon une méthode validée (filtration sur une colonne S400 HR MicroSpin^{™} et dilution dans un milieu glacé). Les dilutions sont transférées dans les unités de culture cellulaire. Après incubation, le titre infectieux est calculé selon la méthode de calcul Spearman-Kärber. La réduction de l'infectivité du virus est calculée et correspond à la différence des titres du virus (témoin viral), exprimés en log, avant et après le traitement avec le produit. Le test a été réalisé sur des souches du coronavirus humain 229E (HCoV-229E).

### Résultats

### 1. Contrôles

### i) Détermination de la cytotoxicité du produit

La cytotoxicité correspond à l'altération morphologique des cellules empêchant la visualisation de l'effet cytopathogène. Après neutralisation, des dilutions successives du produit soumis à l'essai sont mises en contact avec les cellules afin d'évaluer leur cytotoxicité.

**[Tableau 1]**

| Concentration test (%) | Dilutions du filtrat testées | | | |
|---|---|---|---|---|
| 80 | 10⁻¹ | 10⁻² | 10⁻³ | 10⁻⁴ |
| 70 | - | - | - | - |
| 5 | - | - | - | - |

| | | | | |
|---|---|---|---|---|
| - : absence de cytotoxicité (tapis cellulaire intacte) ; C : présence d'un effet cytotoxique. | | | | |

### Conclusion

Après filtration sur colonne MicroSpin^{™} S400 HR, la dilution 10⁻¹ne présente plus d'activité cytotoxique pour les cellules Huh-7 pour le produit à 80 %, à 70 % et à 5 %.

L'extrait de raisin selon l'invention ne présente pas d'effet cytotoxique.

### ii) Détermination de la sensibilité des cellules au virus

Le contrôle d'interférence a pour but de vérifier que la sensibilité des cellules à l'infection virale n'est pas influencée négativement par le traitement avec la solution d'essai du produit. La sensibilité des cellules au virus est appréciée par comparaison de deux titrages de la suspension mère du virus : l'un obtenu sur des cellules traitées 1h avec la plus basse dilution apparemment non cytotoxique du produit, l'autre sur des cellules non traitées.

**[Tableau 2]**

| Concentrations testées (%) | Concentration subcytotoxique (dilution du filtrat) | Titre viral (log TCID₅₀/mL) | | |
|---|---|---|---|---|
| | | Suspension virale cultivée sur cellules traitées à la concentration subcytotoxique | Suspension virale cultivée sur cellules non traitées | Différence de titre viral |
| 80 | 10⁻¹ | 6.6 | 6.8 | 0.1 |
| 70 | 10⁻¹ | 6.4 | | 0.4 |
| 5 | 10⁻¹ | 6.4 | | 0.4 |

La différence entre ces deux titres doit être inférieure ou égale à 1 log afin que l'essai de sensibilité des cellules au virus soit validé.

### Conclusion

Les produits à 80%, 70% et 5% permettent une titration correcte du coronavirus humain 229E.

### iii) Contrôle de l'efficacité de la suppression de l'activité virucide

Ce contrôle permet de vérifier que la filtration sur colonne MicroSpinTM S400 HR est capable d'arrêter l'activité virucide du produit.

**[Tableau 3]**

| Concentrations testées (%) | Titre viral (log TCID₅₀/mL) | | |
|---|---|---|---|
| | Filtration sur colonne MicroSpin^{™} S400 HR puis mise en contact avec la suspension virale (dilution et attente de 30 min sur glace avant titrage) | Suspension virale témoin | Différence de titre viral |
| 80 | 3.6 | 6.8 | 0.1 |
| 70 | 3.5 | | 0.3 |
| 5 3.6 | | | 0.1 |

La différence de titre avec l'essai de suspension doit être ≤ 0.5 log.

### Conclusion

La technique de filtration sur colonne MicroSpin^{™} S400 HR permet bien l'arrêt de l'activité virucide du produit.

### iv) Test d'inactivation virale de référence

Ce contrôle interne de l'essai permet d'évaluer la sensibilité du lot de virus par rapport à un produit de référence : le formaldéhyde à 1,4% (m/V).

**[Tableau 4]**

| | Titre viral (log TCID₅₀/mL) | Réduction du titre viral |
|---|---|---|
| Suspension virale témoin | 6.1 | - |
| Essai d'inactivation après 5 minutes de contact | 4.8 | 1.4 |
| Essai d'inactivation après 15 minutes de contact | ≤ 3.5 | ≥ 2.6 |

La réduction entre le logarithme du titre du témoin viral et celui du virus utilisé lors de l'essai de référence pour l'inactivation des virus enveloppés doit être comprise entre 0.75 et 3.5 log après 5 min et entre 2 et 4 log après 15 min.

Les résultats sont présentés à la Figure 1.

### Conclusion

La sensibilité de la suspension virale utilisée pour l'essai est conforme aux prescriptions de la norme.

### 2. Evaluation de l'activité virucide

### Principe

L'extrait de raisin (à une concentration donnée) est mis en contact avec la suspension virale et la substance interférente. La réaction est stoppée par filtration sur colonne MicroSpin^{™} S400 HR après le temps de contact requis puis dilution à froid. Le titre de chaque essai est déterminé, exprimé en logarithme de Dose Infectieuse CytoToxique capable d'infecter 50% des cellules (Log DICT₅₀), et calculé suivant la méthode Spearman et Kärber, décrite dans l'annexe C de la norme EN 14476 de Juillet 2019. La limite de détection de la technique de titrage correspond au titre minimal théorique résultant de la détection d'une particule virale dans la première dilution testée. La limite de détection est fonction de la raison et du volume testé de cette dilution.

### Résultats

### i. Titre viral

**[Tableau 5]**

| | Titre viral en log TCID₅₀/mL (Intervalle de confiance à 95%) |
|---|---|
| Avant filtration sur colonne MicroSpin^{™} S400 HR | 6.8 (±0.3) |
| Après filtration sur colonne MicroSpin^{™} S400 HR | 6.6 (±0.3) |

La colonne MicroSpin^{™} S400 HR ne réduit pas l'infectivité du virus (intervalles de confiance chevauchants).

**[Tableau 6]**

| | Titre viral en log TCID₅₀/mL dans la solution testée après ... minutes | | |
|---|---|---|---|
| | 0 | 2 | 5 |
| Suspension d'essai de virus | 5.6 (±0.3) | 5.5 (±0) | 5.5 (±0) |

Compte tenu du niveau de cytotoxicité du produit, et de la dilution pour stopper la réaction, la limite de détection est 1.5 log DICT₅₀/mL pour toutes les concentrations. Avec un titre initial à 5.6 log DICT₅₀/mL, l'activité virucide maximale pouvant être quantifiée dans cet essai est de 4.1 log.

**[Tableau 7]**

| Produit | Concentration dans l'essai (%) | Log TCID₅₀/mL dans la solution d'essai après ... minutes | |
|---|---|---|---|
| | | 2 | 5 |
| Extrait de pépins de raisin selon l'invention | 80 | ≤ 1.5 (±0) | ≤ 1.5 (±0) |
| | 70 | 1.9 (±0.4) | 1.9 (±0.4) |
| | 5 | 3.1 (±0.4) | 3.5 (±0) |
| Témoin viral essai | n.a. | 5.6 (±0.3) | 5.6 (±0.3) |

### Ces résultats sont représentés à la Figure 2.

### ii. Réduction

**[Tableau 8]**

| Produit | Concentrations dans l'essai (%) | Réduction (log TCID₅₀/mL) dans l'essai après ... minutes | |
|---|---|---|---|
| | | 2 | 5 |
| Extrait de pépins de raisin selon l'invention | 80 | ≥ 4.1 (±0.3) | ≥ 4.1 (±0.3) |
| | 70 | 3.8 (±0.5) | 3.8 (±0.4) |
| | 5 | 2.5 (±0.4) | 2.1 (±0.3) |

### Conclusion

L'extrait de pépins de raisin selon la présente invention possède une activité virucide supérieure ou égale à 4.1 log à l'encontre de la souche humaine du coronavirus 229E aux concentrations testées pour un temps de contact de 2 ou 5 minutes, en conditions propres.

### 3) Vérification de la méthodologie

Un test est considéré comme valide lorsque les critères suivants sont remplis :
- Le titre de la suspension d'essai est suffisant pour permettre la réduction de 4 log après traitement avec le produit ;
- La réduction entre le logarithme du titre du témoin viral et celui du virus utilisé lors de l'essai de référence pour l'inactivation du coronavirus humain doit être comprise entre 0,75 et 3,5 après 5 min et entre 2 et 4 après 15 min (référence au virus de la vaccine, seul virus enveloppé obligatoire de la norme)
- La cytotoxicité de la solution d'essai du produit n'affecte pas la morphologie et la croissance cellulaire, ni la sensibilité au virus dans les dilutions du mélange d'essai qui sont nécessaires pour démontrer une réduction de 4 log du titre viral
- Lors du contrôle de l'efficacité de l'arrêt de l'activité du produit, la différence de titre avec la suspension d'essai doit être ≤ 0,5 log
- Une concentration au moins par essai doit montrer une réduction de 4 log ou plus et une concentration au moins doit montrer une réduction inférieure à 4 log

Dans le cadre de cet essai, l'ensemble des critères sont remplis.

### Conclusion

L'extrait de pépins de raisin selon la présente invention possède avantageusement une activité virucide à l'encontre des virus de la famille des coronavirus. L'extrait de pépins de raisin selon l'invention est avantageusement efficace à l'encontre des virus de la famille des coronavirus et est non cytotoxique. L'extrait de pépins de raisin selon la présente invention permet donc d'offrir des désinfectants et antiseptiques d'origine végétale ayant un faible impact sur la santé et l'environnement.

### Exemple 2 : Effet activateur de l'extrait de raisin selon l'invention

Deux formules en phase aqueuse ont été testées pour leur efficacité contre le betacoronavirus :
- une solution aqueuse comprenant 7% de DDAC
- une solution aqueuse dite activée comprenant 1% de DDAC et 1% d'un extrait de raisin.

Les résultats, obtenus selon la méthodologie présentée ci-avant de la norme EN14476, sont présentés dans le tableau ci-après afin de détecter la dose efficace en DDAC ayant une activité virucide sur le betacoronavirus.

**[Tableau 9]**

| Conditions | Formule Standard | | Formule suractivée | |
|---|---|---|---|---|
| Température = 20°C Temps de contact = 5 min | Dose efficace DDAC | 0,07% (dilution formule dans eau 1%) | Dose efficace DDAC | 0,007% (dilution formule dans eau 0.7%) |
| conditions de propreté | Dose cytotoxique DDAC | 0,14% (dilution formule dans eau 2%) | Dose cytotoxique DDAC | 0,035% (dilution formule dans eau 3.5%) |
| Température = 20°C Temps de contact = 5 min conditions de saleté | Dose efficace DDAC | 0,07% (dilution formule dans eau 1%) | Dose efficace DDAC | 0,01% (dilution formule dans eau 1%) |
| | Dose cytotoxique DDAC | 0,14% (dilution formule dans eau 2%) | Dose cytotoxique DDAC | 0,035% (dilution formule dans eau 3.5%) |

Avantageusement, l'extrait de raisin selon l'invention est un activateur de la DDAC et permet de diminuer la dose efficace et utile de DDAC d'un facteur 10, et ce tel que représenté à la Figure 3.

### Exemple 3 : Composition désinfectante

Dans cet exemple, une composition désinfectante est reproduite dans le tableau 10 ci-après.

**[tableau 10]**

| Ingrédients | Quantité (en pourcentage %) |
|---|---|
| Solvant biosourcé issu du glycérol | 20 |
| DDAC | 1 |
| Extrait de raisin | 1 |
| Alcool ethoxylé biosourcé | 2.7 |
| Agent tensioactif biosourcé tel que le polysorbate | 1.8 |
| Eau | qsp |

La composition pourra être appliquée sur une surface ou un textile ou servir à immerger un instrument ou un textile dans un procédé de désinfection.

### Exemple 4 : Composition antiseptique sous forme de gel

Dans cet exemple, une composition antiseptique sous forme de gel hydroalcoolique est reproduite dans le tableau 11 ci-après.

**[tableau 11]**

| Ingrédients | Quantité (en pourcentage %) |
|---|---|
| Eau | qsp |
| Alcool | 10 |
| Glycérine | 1.5 |
| Acrylates/C10-30 alkyl acrylate crosspolymer neutralisé | 0.5 |
| Extrait de raisin | 3 |

Typiquement, la composition antiseptique pourra être utilisée pour le traitement hygiénique des mains par friction ou le lavage hygiénique des mains.

### Exemple 5 : Composition antiseptique sous forme de crème

Dans cet exemple, une composition antiseptique sous forme de crème est reproduite dans le tableau 12 ci-après.

**[tableau 12]**

| Ingrédients | Quantité (en pourcentage %) |
|---|---|
| Eau | qsp |
| Emulsifiant | 3.5 |
| Cire | 3.0 |
| Agent épaississant | 1.0 |
| Isostearyl isostearate | 4.0 |
| Huile minérale | 3.0 |
| Glycérine | 4.0 |
| Extrait de raisin | 3 |

Typiquement, la crème pourra être utilisée pour le soin des mains abimées par exemple par des lavages fréquents. La crème antiseptique possède en effet un pouvoir antiseptique et avantageusement un pouvoir réparateur par la présence de ces agents hydratants.

### Exemple 6 : démonstration de l'activité virucide d'un extrait de pépins de raisin selon l'invention à l'encontre des virus de la famille des adénovirus, des norovirus murins, du virus de la vaccine et des poliovirus.

L'activité virucide de l'extrait de pépins de raisin selon l'invention a été évalué selon les critères de la norme EN14476 :2019 tel que précédemment décrit.

Produits testés :

| Dilution | Préparation |
|---|---|
| 97% | Produit en tant que tel |
| 80% | Produit en tant que tel |
| 5% | Solution à 6.25% (v/v) dans de l'eau distillée |

Conditions du test

| Souches testées (standard) | Lignées cellulaires pour la propagation des souches testées |
|---|---|
| Adénovirus type 5, souche Adénoïde, 75 ATCC VR-5 | HeLa, ATCC CCL.2 |
| Norovirus murin MNV, souche 599 Berlin Friedrich Loeffler Institute RVB-0651 | Raw 264.7 ATCC TIB-71 |
| Poliovirus type 1 LSc-2ab, Eurovir Hygiene Institut | LLC-MK2 ATCC CCL-7.1 |
| Virus de la vaccine, souche Ankara, ATCC-VR-1508 | BHK-21-cl 13, IZS-Brescia |
| | |
| Temps de contact | 5min ± 10 secondes |
| Température du test | 20°C ± 1°C |
| Substance interférente | Albumine bovine 0.3 g/l (conditions propres) |
| Conditions d'incubation | 37°C ± 1°C, 5% CO₂ |

### Matériels et Réactifs :

Milieu de croissance : MEM 10% FCS
Milieu de croissance : MEM 2% FCS

Selon les critères de la norme EN 14476/2019, l'extrait de raisin selon l'invention a une activité virucide (log R ≥ 4) lorsqu'il est utilisé à 97% et à 80% à l'encontre des adénovirus, des norovirus murins, du virus de la vaccine et des poliovirus. Selon cette même norme, une activité virucide peut donc être revendiquée.

### Exemple 6 : démonstration de l'effet antiseptique d'une composition selon l'invention

Le pouvoir antiseptique d'une crème comprenant un extrait selon l'invention a été évalué et comparé à la même crème ne comprenant pas l'extrait selon l'invention. Les compositions des crèmes sont décrites ci-après :

| Ingrédient | Crème comprenant l'extrait selon l'invention | Témoin |
|---|---|---|
| Extrait de pépins de raisin | 4% | 0% |
| Caprylic/ Capric Triglyceride (Émollient) | 10% | 10% |
| Cetearyl Alcohol (and) Dicetyl Phosphate (and) Ceteth-10 Phosphate (Emulsionnant H/E) | 5% | 5% |
| Glycérine (agent hydratant) | 5.5% | 5.5% |
| Gomme xanthane (épaississant) | 0.5% | 0.5% |
| NaOH pour ajuster le pH à 5 | 0.3% | 0.3% |
| Eau | Qsp 100% | Qsp 100% |

### Le protocole est le suivant :

Un échantillon de 1 gramme de chaque produit (crème avec extrait et crème sans extrait) est préalablement sorti de son contenant et placé dans des pots stériles, en conditions aseptiques, trois répétitions sont réalisées par produit.

Chaque produit est contaminé artificiellement par la même suspension mère de virus par dépôt de 100 µL en microgouttelettes avec une concentration de virus de 1.10⁷ DICT50/mL.

A la fin du temps de contact de 2 minutes ou 30 minutes, chaque produit est mélangé à un neutralisant universel afin de stopper l'action du principe actif. L'ensemble est agité avec des billes de verre pendant 1 min à l'aide d'un vortex, puis incubé pendant 30 minutes à température ambiante pour laisser le temps d'action du neutralisant. A l'issue des 30 minutes de neutralisation, chaque mélange est placé dans un sachet d'homogénéisation Stomacher^{®} avec filtre latéral stérile pour permettre de récupérer la solution de virus à analyser débarrassée des débris de produit et centrifugé 10 minutes pour éliminer les débris restants.

Après centrifugation, le surnageant est récupéré puis filtré sur un filtre de porosité 0,20 µm. Après filtration, la solution contenant les virus est passée sur colonne MicrospinTM afin de retirer la cytotoxicité induite par la crème. Après passage sur colonne MicrcospinTM, la solution est concentrée sur colonne Amicon^{®}. Les virus sont ensuite quantifiés sur cellules permissives.

Les critères d'efficacité sont les mêmes que ceux de l'EN14476.

Avantageusement, la crème comprenant l'extrait selon l'invention permet la diminution de l'activité virucide, comparativement à la crème témoin, permettant de conclure au pouvoir antiseptique de l'extrait et de la crème comprenant l'extrait selon l'invention.

## Revendications

1. Utilisation d'un extrait de raisin en tant que désinfectant à l'encontre des virus choisis parmi les virus de la famille des coronavirus, des adénovirus, des norovirus murins, des virus de la vaccine et des poliovirus.

2. Utilisation selon la revendication 1 **caractérisée en ce que** le désinfectant est désinfectant à l'encontre des virus de la famille des coronavirus.

3. Utilisation selon la revendication 2 **caractérisée en ce que** l'extrait est en outre désinfectant à l'encontre des virus de la famille des adénovirus, des norovirus murins, des virus de la vaccine et des poliovirus.

4. Utilisation selon l'une quelconque des revendications 1 à 3, **caractérisée en ce que** l'extrait de raisin est un extrait de pépins de l'espèce *Vitis vinifera L.*

5. Procédé de désinfection d'une surface, d'un instrument ou d'un textile au moyen d'une composition désinfectante comprenant un extrait de raisin, le procédé comprenant les étapes consistant à appliquer ladite composition sur ladite surface ou ledit textile ou à immerger ledit instrument ou ledit textile dans ladite composition.

6. Procédé de désinfection selon la revendication 5, **caractérisé en ce que** la composition désinfectante comprend au moins 0.2% en poids par rapport au poids total de la composition d'un extrait de pépins de raisin.

7. Procédé de désinfection selon la revendication 5ou 6, **caractérisé en ce que** la composition désinfectante comprend en outre, au moins un ammonium quaternaire ou un sel d'ammonium quaternaire choisi parmi le chlorure d'alkyldiméthylbenzylammonium (ADBAC, BKC), le cation tétraméthylammonium (TMAC), le N-(3-aminopropyl)-N-dodecylpropane-1,3-diamine (diamine), le propionate de N,N-Didecyl-N-methyl-poly(oxyethyl) ammonium (Bardap26), le chlorure de didécyldiméthylammonium (DDAC).

8. Procédé d'antisepsie non thérapeutique, **caractérisé en ce qu'**il comprend l'application sur les matières kératiniques d'une composition antiseptique comprenant, dans un milieu physiologiquement acceptable, un extrait de raisin.

9. Composition antiseptique comprenant, dans un milieu physiologiquement acceptable, un extrait de raisin, pour son utilisation thérapeutique en tant qu'antiseptique.

10. Composition antiseptique selon les revendications 8 ou 9, **caractérisée en ce que** ladite composition est sous forme de solution monophasique aqueuse, sous forme de solution monophasique hydroalcoolique, sous forme de gel, hydroalcoolique ou aqueux, ou sous forme d'émulsion.

11. Composition désinfectante comprenant un extrait de raisin et un agent désinfectant choisi parmi un ammonium quaternaire, un sel d'ammonium quaternaire, une amine, un aldéhyde, un phénol, un alcool, l'acide peracétique ou leurs mélanges, ledit extrait de raisin étant présent en un teneur d'au moins 1% en poids par rapport au poids total de la composition et l'agent désinfectant étant présent en une teneur d'au moins 1% en poids par rapport au poids total de la composition.

12. Composition désinfectante selon la revendication 11 **caractérisée en ce que** l'agent désinfectant est un ammonium quaternaire et/ou un sel d'ammonium quaternaire, choisi parmi le chlorure d'alkyldiméthylbenzylammonium (ADBAC, BKC), le cation tétraméthylammonium (TMAC), le N-(3-aminopropyl)-N-dodecylpropane-1,3-diamine (diamine), le propionate de N,N-Didecyl-N-methyl-poly(oxyethyl) ammonium (Bardap26), le chlorure de didécyldiméthylammonium (DDAC) ou leur mélange.
